# EUROPÄISCHE PATENTSCHRIFT

(11) **EP 2 316 389 B1**
(45) Veröffentlichungstag und Bekanntmachung des Hinweises auf die Patenterteilung: **11.09.2013**
(21) Anmeldenummer: 09013410.7
(22) Anmeldetag: 23.10.2009
(51) Int. Cl.: A61F 2/50, A61F 2/60, A61F 2/64, A61F 2/58, A61F 2/74

(54) **Hydraulikdämpfer fur prothetische und orthopädische Vorrichtungen**
Hydraulic dampeners for prosthetic and orthopaedic devices
Amortisseur hydraulique pour dispositifs prothétiques et orthopédiques

(43) Veröffentlichungstag der Anmeldung: 04.05.2011
(73) Patentinhaber: Streifeneder ortho.production GmbH, 82275 Emmering (DE)
(72) Erfinder: Krafczyk, Thomas, 82140 Olching (DE); Osgyan, Rudolf, 93049 Regensburg (DE); Tischler, Helmut, 93053 Regensburg (DE); Weber, Wolfgang, 82256 Fürstenfeldbruck (DE)
(74) Vertreter: Bosch, Matthias

(56) Entgegenhaltungen:
- GB-A- 2 338 653
- US-A- 4 662 486
- US-A- 5 092 902
- US-A- 5 376 135
- US-A- 6 106 560
- US-A1- 2008 228 287

## Beschreibung

Gegenstand der vorliegenden Erfindung ist ein Hydraulikdämpfer für prothetische und/oder orthopädische Vorrichtungen, insbesondere zur Anwendung in einem künstlichen Kniegelenk.

Aus dem Stand der Technik, bspw. US 2008/0228287 sind künstliche Kniegelenke bekannt, deren Beuge- und Streckfunktion über ein Hydraulikdämpferelement realisiert werden kann. Hierzu befindet sich in diesen Hydraulikdämpfern ein Kolben in einem Zylindergehäuse, wobei der Kolben über ein Hydraulikfluid in die beiden möglichen Bewegungsrichtungen des Zylindergehäuses bewegbar ist. Um die gewünschte gedämpfte Bewegung des Kolbens zu ermöglichen, strömt das Hydraulikfluid auf beiden Seiten des Kolbens durch entsprechende Kanäle oder durch Bohrungen im Kolben. In beiden Fällen richtet sich die gedämpfte Bewegung des Kolbens nach der Durchflussleistung des Hydraulikfluids, die sich ihrerseits aus der Viskosität des Hydraulikfluids und den Durchflussdurchmessern bemisst.

Bei Hydraulikdämpfern, die für prothetische Vorrichtungen verwendet werden, ist es grundsätzlich das Ziel, die substituierte Bewegung, bspw. eines amputierten Gliedmaßes möglichst realistisch nachzuempfinden. Aufgrund des komplexen Bewegungsablaufs eines natürlichen Gliedmaßes, bspw. bei der Beugung oder Streckung, ist es für Patienten, die bspw. das Laufen mit einem künstlichen Gliedmaß neu erlernen müssen, ein durchaus langwieriger Lernprozeß, den nunmehr künstlich eingeleiteten und durchgeführten Beuge- und Streckprozess zu akzeptieren und die jeweilige Bewegung noch vorhandenen natürlichen Gliedmaßes darauf abzustimmen.

Dieser Lernprozess wird herkömmlicherweise durch künstliche Gliedmaßen, bspw. Unterschenkelprothesen mit Kniegelenk, realisiert, die unterschiedliche Dämpfungsverhalten aufweisen. Die Umstellung und Einstellung eines Patienten auf ein neues künstliches Gliedmaß ist indes ein Aufwand, der sowohl logistische als auch für den Patienten nachteilige psychologische Auswirkungen hat. Denn ein durch eine Amputation traumatisierter Patient wird sich mit einem neuen Gliedmaß nur sehr langsam identifizieren, sodass ein gerade in der postoperativen Lernphase häufig auszutauschendes Gliedmaß zu psychologischen Belastungen führen kann.

Aufgabe der vorliegenden Erfindung ist es daher, einen Hydraulikdämpfer für prothetische und/oder orthopädische Vorrichtungen bereitzustellen, mit dem es möglich ist, jeweilige Trainings- und Lernzustände kontinuierlich an die Anforderungen anzupassen und ein dem natürlichen Bewegungsablauf möglichst ähnliches Dämpfungsverhalten nachzuempfinden.

Diese technische Aufgabe wird durch den erfindungsgemäßen Hydraulikdämpfer mit den Merkmalen gemäß Anspruch 1 gelöst. Vorteilhafte Weiterbildungen der vorliegenden Erfindung sind in den Unteransprüchen 2 bis 8 wiedergegeben.

Die orthopädische Vorrichtung besteht aus einem Kolben, der in einem Zylindergehäuse durch ein Hydraulikfluid beweglich angeordnet ist, wobei das Zylindergehäuse aufweist:
- einen ersten Kanal, durch den ein Hydraulikfluid strömt und eine Kolbenbewegung in die eine Richtung E veranlasst,
- einen zweiten Kanal, durch den das Hydraulikfluid strömt und eine Kolbenbewegung in die andere Richtung F veranlasst,
- sowie einen dritten Kanal, durch den das Hydraulikfluid strömt und die Kolbenbewegung in der einen oder anderen Richtung E oder F dämpft,
wobei der Kolben so dimensioniert ist, dass sowohl der erste Kanal als auch der dritte Kanal mit dem Kolben verschließbar sind.

Vorteilhafterweise ist im ersten und/oder zweiten Kanal ein Drosselventil angeordnet.

Vorteilhafterweise ist der dritte Kanal mit dem ersten Kanal verbunden; zweckmäßigerweise erfolgt dies über eine Bohrung, die vom ersten Kanal in den dritten Kanal läuft und erfindungsgemäß ein Drosselventil aufweist.

Vorteilhafterweise ist das Drosselventil im dritten Kanal manuell einstellbar und zweckmäßigerweise als Stellschraube ausgebildet, die an ihrem einen Ende eine Dichtfläche aufweist, die so ausgebildet ist, dass der dritte Kanal vollständig geschlossen werden kann.

Erfindungsgemäß ist im ersten und zweiten Kanal jeweils ein Rückschlagventil angeordnet, um eine bestimmungsgemäße Fließrichtung des Hydraulikfluids, das zweckmäßigerweise ein Hydrauliköl ist, zu gewährleisten.

Erfindungsgemäß ist der erste Kanal als Bypasskanal zum Zylindergehäuse ausgebildet. Erfindungsgemäß ist der dritte Kanal so ausgebildet, dass er vom Zylindergehäuse in den ersten Kanal verläuft und ein Drosselventil aufweist. Erfindungsgemäß ist der zweite Kanal ebenfalls als Bypasskanal ausgebildet, der parallel zum Zylindergehäuse verläuft.

Wesentlicher Vorteil der vorliegenden Erfindung bei der Verwendung des erfindungsgemäßen Hydraulikdämpfers für prothetische und/oder orthopädische Vorrichtungen ist es, dass die Bewegung des Kolbens in einem ersten Zeit- und Bereichsabschnitt gemäß der vorbestimmten Dimensionierung des ersten Kanals erfolgt und nach Erreichen dieses Zeit- und Bereichsabschnitts eine manuell einstellbare gedämpfte Bewegung eingeleitet wird. Insbesondere im orthopädischen Anwendungsbereich ist es von besonderem Vorteil, in einem bestimmten Bewegungsabschnitt die Dämpfung manuell einstellen zu können, um bspw. den Endanschlag einer bestimmten Gelenkvorrichtung definiert zu dämpfen, sodass der Patient eine entsprechende Einstellung nach Leistungsgrad einstellen kann.

Bei einer bevorzugten Ausführungsform der vorliegenden Erfindung wird die Drosselschraube manuell oder mittels Werkzeug so eingestellt, dass die Dämpfung im Randbereich bspw. bei der Extension stärker erfolgt. Dies kann bspw. zu einer zusätzlichen Dämpfung in den letzten 5 % bis 10 % des Kolbenhubs führen. Im Laufe des Lernprozesses kann der Patient die Dämpfung individuell anpassen, wobei jedoch auch eine Anpassung an unterschiedlich Aktivitäten sinnvoll sein kann. Es kann erforderlich sein, die Dämpfung bei der Ausübung einer Sportart, bspw. dem Radfahren wesentlich zu verändern, ebenso kann dies bei der Bewältigung von Treppen oder dem Joggen der Fall sein. Erfindungsgemäß kann die Dämpfung im Endlagenbereich der Extension zu jederzeit eingestellt werden.

Ein weiterer Vorteil der vorliegenden Erfindung ist die Vermeidung von unangenehmen metallenen Auflaufgeräuschen bei der Extension. Durch die erfindungsgemäße Dämpfung wird kurz vor der Kontaktaufnahme der Gelenkbereich die Geschwindigkeit so stark reduziert, dass bei Kontakt der beiden Gelenkbereiche kein wesentliches Geräusch entsteht.

Der erfindungsgemäße Hydraulikdämpfer wird als hydraulische Extensionsendlagedämpfung in einem Prothesenkniegelenk eingesetzt. Ebenso ist es denkbar, dass der erfindungsgemäße Hydraulikdämpfer in einem Ellenbogengelenk eingesetzt wird oder in anderen prothetischen oder orthopädischen Vorrichtungen, wie bspw. einer Prothese zur Unterstützung und Entlastung des noch bestehenden natürlichen Kniegelenks.

Eine bevorzugte Ausführungsform der vorliegenden Erfindung wird anhand von Figuren näher erläutert. Es zeigen:
Figur 1 einen Längsquerschnitt durch den erfindungsgemäßen Hydraulikdämpfer;
Figur 2 einen Längsquerschnitt durch den erfindungsgemäßen Hydraulikdämpfer gemäß Figur 1 bei einer Flexionsbewegung;
Figur 3 einen Längsquerschnitt durch den erfindungsgemäßen Hydraulikdämpfers bei einer Extensionsbewegung im ersten Abschnitt;
Figur 4 einen Längsquerschnitt durch den erfindungsgemäßen Hydraulikdämpfers im zweiten Abschnitt einer Extensionsbewegung.

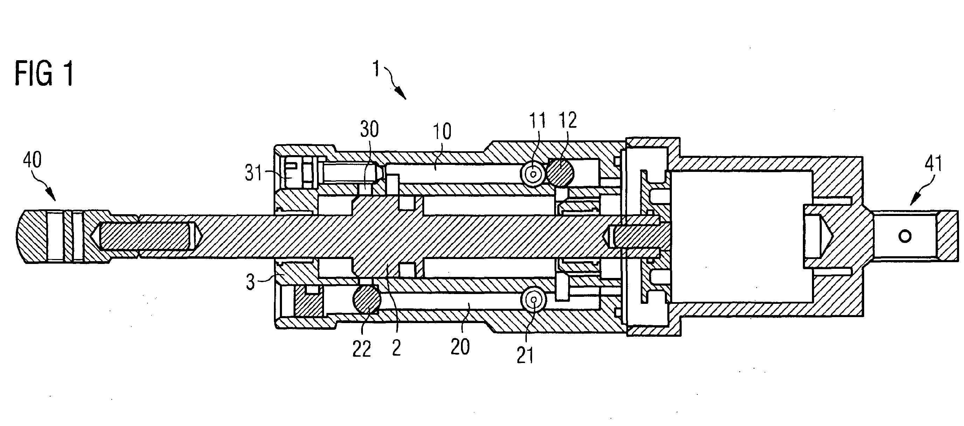

Figur 1 zeigt einen erfindungsgemäßen Hydraulikdämpfer 1 für prothetische und/oder orthopädische Vorrichtungen, insbesondere für ein künstliches Kniegelenk, wobei der Anschluss im proximalen Bereich über eine Einrichtung 40 und im distalen Bereich über eine Einrichtung 41 erfolgt.

Der erfindungsgemäße Hydraulikdämpfer besteht aus einem Kolben 2, der mit einer Kolbenstange verbunden ist und auf der proximalen Seite den Anschluss 40 aufweist. Der Kolben 2 ist zweckmäßigerweise kreisförmig ausgebildet und ruht in einem Zylindergehäuse 3, das über herkömmliche Dichtungen (nicht dargestellt) gegenüber der Atmosphäre abgedichtet ist. Der Raum zwischen Kolben 2, Kolbenstange und Zylindergehäuse 3 ist durch ein nicht dargestelltes Hydraulikfluid, vorzugsweise ein Hydrauliköl, gefüllt, welches bei der Translationsbewegung des Kolbens 2 zirkuliert. Zweckmäßigerweise durchläuft der Kolben 2 zwei Endlagen, in deren Nähe die Ein- und Austrittsöffnungen des ersten und zweiten Kanals 10, 20 angeordnet sind. Gemäß Figur 1 ist ein dritter Kanal 30 im Zylindergehäuse 3 angeordnet, der in Verlängerung des ersten Kanals 10 angeordnet ist und mit diesem in Wirkverbindung steht. Zum Öffnen und Schließen des dritten Kanals 30 ist ein Drosselventil 31 vorgesehen, das gemäß Figur 1 als Stellschraube ausgebildet ist, die an ihrem Ende eine Dichtfläche aufweist, um den dritten Kanal 30 gegenüber dem ersten Kanal 10 gegebenenfalls teilweise oder vollständig abzudichten.

Bei einer bevorzugten Ausführungsform der vorliegenden Erfindung ist es möglich, mittels Drosselventil 31 den Querschnitt des dritten Kanals zwischen 0 und 3,5 mm einzustellen, sodass die wirksame Durchflussfläche stufenlos von 0 mm² bis ca. 16,8 mm² einstellbar ist. Ebenso ist es möglich, andere Querschnittsflächen zu bestimmen.

Erfindungsgemäß befindet sich der Eintritt des dritten Kanals 30 zur Realisierung der Extensionsendlagendämpfung im Randbereich des Zylindergehäuses.

Gemäß Figur 1 wird der Austritt des dritten Kanals 30 im letzten Sechstel des Kolbenhubs angeordnet, sodass bis zum Erreichen dieses letzten Sechstels die Bewegung des Kolbens 2 ausschließlich über die Verdrängung des Hydraulikfluids in den ersten Kanal 10 erfolgt und erst im letzten Sechstel des Kolbenhubs die Wirkung des dritten Kanals einsetzt. Der Durchflussquerschnitt des ersten Kanals wird über ein Drosselventil 11 eingestellt und ein Rückfluss in den ersten Kanal wird über ein Rückschlagventil 12 bewerkstelligt. Die gleichen Funktionselemente finden sich auch im zweiten Kanal wieder, sodass auch dort ein Drosselventil 21 und ein Rückschlagventil 22 angeordnet ist.

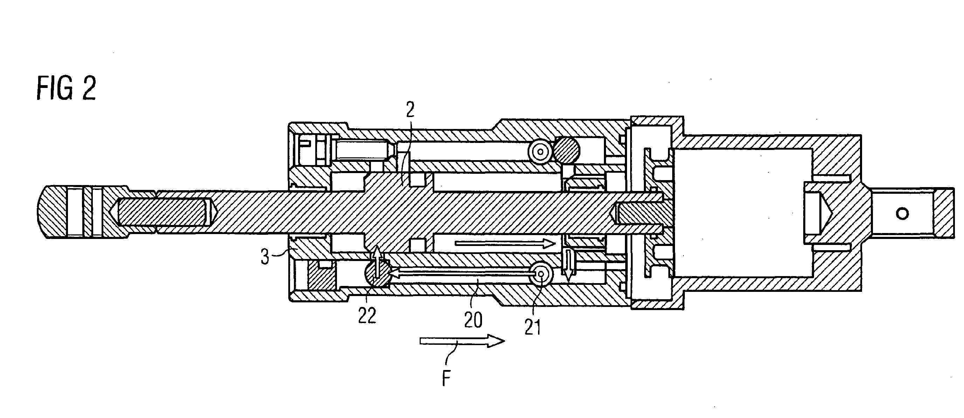

Gemäß Figur 2 erfolgt die Flexionsbewegung durch eine Bewegung des Kolbens 2 in Richtung des dargestellten Pfeils F, wobei gemäß dargestellter Pfeile das Hydraulikfluid aus dem Zylindergehäuse 3 in den zweiten Kanal 20 verdrängt wird und gegebenenfalls über das Drosselventil 21 einstellbar ist. Ein Rückfließen des Hydraulikfluids wird über das Rückschlagventil 22 verhindert.

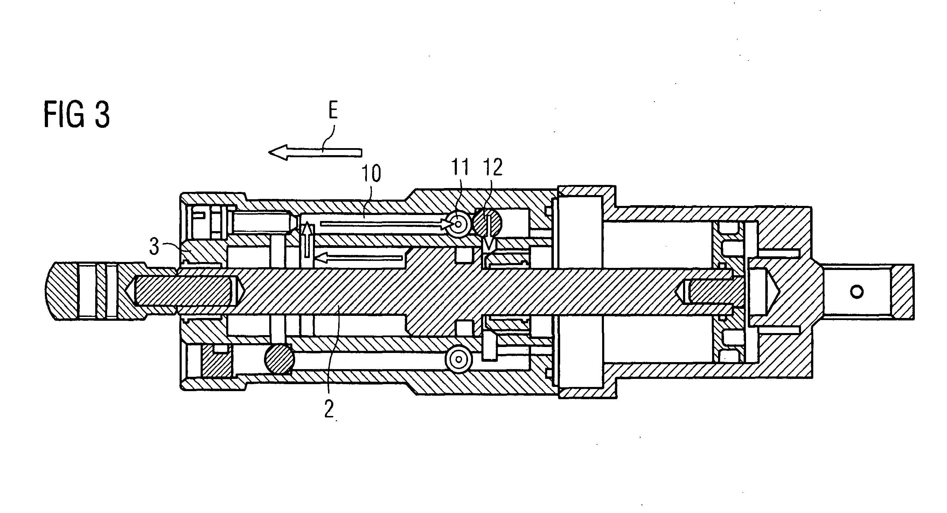

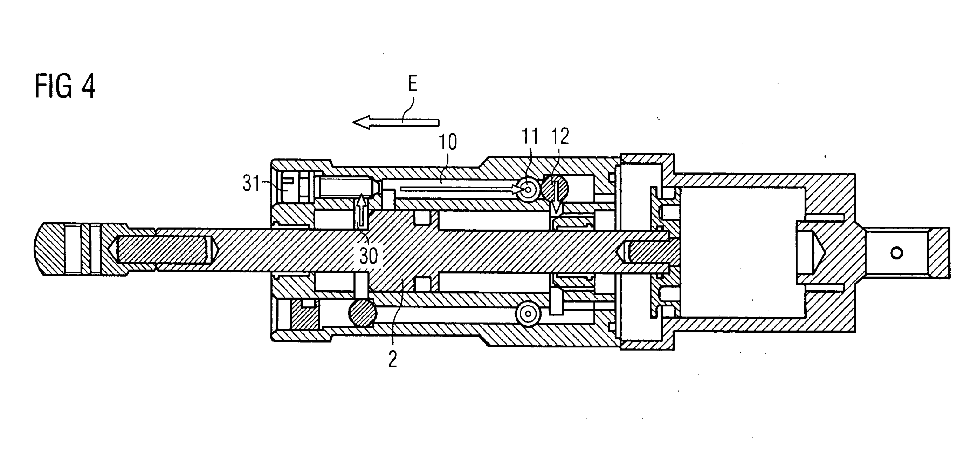

In der gegenläufigen Extensionsbewegung, die mit E und dementsprechenden Pfeil dargestellt ist, wird gemäß Figur 3 das Hydraulikfluid gemäß Pfeilrichtung aus dem Zylindergehäuse 3 verdrängt und durch den ersten Kanal 10 mit Drosselventil 11 und Rückschlagventil 12 gedrückt. Gemäß Figur 4 wird bei Erreichen des dritten Kanals 30 der erste Kanal 10 verschlossen und das Hydraulikfluid kann ausschließlich über den dritten Kanal 30 und das Drosselventil 31 in den ersten Kanal 10 mit dessen Drosselventil 11 und Rückschlagventil 12 einströmen. Aufgrund des verminderten Querschnitts in dem Drosselventil 31 wird die Bewegung des Kolbens 2 in Extensionsrichtung wesentlich reduziert, d.h. gedämpft.

Erfindungsgemäß ist es vorgesehen, bei komplexeren Bewegungsabläufen in der Prothetik oder Orthopädie eine gewünschte Dämpfung durch entsprechende Anordnung weiterer Kanäle und Drosselventil zu erzielen. Dabei kann es erfindungsgemäß notwendig sein, die weiteren Kanäle kaskadenartig mit einander zu verbinden und die Drosselventil stufenweise aufeinander abzustimmen.

## Patentansprüche

1. Hydraulikdämpfer (1) für prothetische und/oder orthopädische Vorrichtungen bestehend aus einem Kolben (2), der in einem Zylindergehäuse (3) durch ein Hydraulikfluid beweglich angeordnet ist, wobei das Zylindergehäuse (3) aufweist:
- einen ersten Kanal (10) durch den ein Hydraulikfluid strömt und eine Kolbenbewegung in die eine Richtung (E) veranlaßt,
- einen zweiten Kanal (20) durch den das Hydraulikfluid strömt und eine Kolbenbewegung in die andere Richtung (F) veranlaßt,
- einen dritten Kanal (30) durch den das Hydraulikfluid strömt und die Kolbenbewegung in der einen oder anderen Richtung (E; F) dämpft, **dadurch gekennzeichnet, dass** der Kolben (2) so dimensioniert ist, dass sowohl der erste Kanal (10) als auch der dritte Kanal (30) mit dem Kolben (2) verschließbar sind.

2. Hydraulikdämpfer gemäß Anspruch 1, wobei im ersten Kanal (10) und/oder zweiten Kanal (20) jeweils ein Drosselventil (11; 21) angeordnet ist.

3. Hydraulikdämpfer gemäß Anspruch 1, wobei der dritte Kanal (30) mit dem ersten Kanal (10) verbunden ist.

4. Hydraulikdämpfer gemäß Anspruch 3, wobei die Verbindung des dritten Kanals (30) mit dem ersten Kanal (10) mittels Drosselventil (31) erfolgt.

5. Hydraulikdämpfer gemäß Anspruch 4, wobei das Drosselventil (31) im dritten Kanal manuell einstellbar ist.

6. Hydraulikdämpfer gemäß einem der vorherigen Ansprüche, wobei im ersten Kanal (10) und zweiten Kanal (20) jeweils ein Rückschlagventil (12; 22) angeordnet ist.

7. Hydraulikdämpfer nach Anspruch 1, wobei der Kolben (12) so dimensioniert ist, dass nach dem Verschluß des ersten Kanals (10), das Hydraulikfluid nur durch den dritten Kanal (30) und das Drosselventil (31) strömt.

8. Künstliches Kniegelenk mit einem Hydraulikdämpfer nach einem der vorherigen Ansprüche.

9. Künstliches Ellenbogengelenk mit einem Hydraulikdämpfer nach einem der Ansprüche 1 bis 7.

## Claims

1. A hydraulic damper (1) for prosthetic and/or orthopedic devices, consisting of a piston (2) arranged to be moved in a cylinder housing (3) by a hydraulic fluid, wherein the cylinder housing (3) comprises:
- a first channel (10) through which a hydraulic fluid flows and causes a piston movement in the one direction (E),
- a second channel (20) through which the hydraulic fluid flows and causes a piston movement in the other direction (F),
- a third channel (30) through which the hydraulic fluid flows and dampens the piston movement in the one or the other direction (E; F),
**characterized in that** the piston (2) is dimensioned such that both the first channel (10) and the third channel (30) are adapted to be closed with the piston (2).

2. The hydraulic damper according to claim 1, wherein a throttle valve (11; 21) each is arranged in the first channel (10) and/or the second channel (20).

3. The hydraulic damper according to claim 1, wherein the third channel (30) is connected with the first channel (10).

4. The hydraulic damper according to claim 3, wherein the connection of the third channel (30) with the first channel (10) is performed by means of a throttle valve (31).

5. The hydraulic damper according to claim 4, wherein the throttle valve (31) is adjustable manually in the third channel.

6. The hydraulic damper according to any of the preceding claims, wherein a check valve (12; 22) each is arranged in the first channel (10) and the second channel (20).

7. The hydraulic damper according to claim 1, wherein the piston (12) is dimensioned such that after closure of the first channel (10) the hydraulic fluid only flows through the third channel (30) and the throttle valve (31).

8. An artificial knee joint comprising a hydraulic damper according to any of the preceding claims.

9. An artificial elbow joint comprising a hydraulic damper according to any of claims 1 to 7.

## Revendications

1. Amortisseur hydraulique (1) pour des dispositifs prothétiques et/ou orthopédiques, constitué d'un piston (2) agencé dans un carter de cylindre (3) en y étant mobile sous l'effet d'un fluide hydraulique, le carter de cylindre (3) comprenant :
- un premier canal (10) à travers lequel s'écoule un fluide hydraulique en engendrant un mouvement du piston dans une direction (E),
- un deuxième canal (20) à travers lequel s'écoule le fluide hydraulique en engendrant un mouvement du piston dans l'autre direction (F),
- drosselun troisième canal (30) à travers lequel s'écoule le fluide hydraulique en amortissant le mouvement du piston dans l'une ou dans l'autre direction (E ; F),
**caractérisé en ce que** le piston (2) est dimensionné de façon à ce qu'aussi bien le premier canal (10) que le troisième canal (30) puissent être obturés par le piston (2).

2. Amortisseur hydraulique selon la revendication 1, dans lequel une valve d'étranglement (11 ; 21) est agencée respectivement dans le premier canal (10) et/ou dans le deuxième canal (20).

3. Amortisseur hydraulique selon la revendication 1, dans lequel le troisième canal (30) est relié au premier canal (10).

4. Amortisseur hydraulique selon la revendication 3, dans lequel la liaison du troisième canal (30) au premier canal (10) s'effectue au moyen d'une valve d'étranglement (31).

5. Amortisseur hydraulique selon la revendication 4, dans lequel la valve d'étranglement (31) située dans le troisième canal peut être réglée manuellement.

6. Amortisseur hydraulique selon l'une des revendications précédentes, dans lequel une valve anti-retour (12 ; 22) est respectivement agencée dans le premier canal (10) et dans le deuxième canal (20).

7. Amortisseur hydraulique selon la revendication 1, dans lequel le piston (12) est dimensionné de manière telle qu'après l'obturation du premier canal (10), le fluide hydraulique s'écoule uniquement à travers le troisième canal (30) et la valve d'étranglement (31).

8. Articulation artificielle du genou comprenant un amortisseur hydraulique selon l'une des revendications précédentes.

9. Articulation artificielle du coude comprenant un amortisseur hydraulique selon l'une des revendications 1 à 7.
